# EUROPEAN PATENT APPLICATION

(11) **EP 0 904 740 A2**
(43) Date of publication of application: **31.03.1999**
(21) Application number: 98850143.3
(22) Date of filing: 17.09.1998
(51) Int. Cl.: A61B 17/80

(54) **Implant with bone screw insert**

(30) Priority: 29.09.1997 US 940022
(71) Applicant: OLERUD, Sven, 740 11 Länna (SE)
(72) Inventor: OLERUD, Sven, 740 11 Länna (SE)
(74) Representative: Henningsson, Gunnar

(57) **Abstract**

The present invention relates to an implant device of the type comprising an implant element (101) and an insert element (109) which is translatorily movable in the implant element, said insert element having a bone screw hole (116) adapted to receive a bone screw which is insertable and lockable therein, and movement of the insert element (109) in relation to the implant element (101) giving adaption of the position of the bone screw hole (116) in relation to the implant element (101).

The invention is characterized by a rotatably arranged adjusting means (170) and transmission means (112, 176) adapted to transmit a rotary motion of the adjusting means (170) to a translatory motion of the insert element (109) in relation to the implant element (101).

## Description

### Field of the Invention

The present invention relates to an implant device of the type comprising an implant element and an insert element which is translatorily movable in the implant element, said insert element having a bone screw hole adapted to receive a bone screw which is insertable and lockable therein, and the movement of the insert element in relation to the implant element giving adaption of the position of the bone screw hole in relation to the implant element.

### Background Art

Implant devices of the above-mentioned type are often used in connection with orthopedic surgery, such as for stabilizing of fractured bones, for stabilizing the position of vertebrae, for reducing of vertebrae etc.

It is desirable that the implant device be flexible in respect of the possibility of adjusting the direction and position of the bone screws relative to each other and relative to the implant element on the one hand in the original fixing of the implant device and, on the other hand, in subsequent adjusting of the shape and fixing of the implant device.

According to the International Patent Application WO 95/35067 an implant plate element is provided with plate parts which can be offset in relation to the implant plate element.

The possibility of displacing the position of the plate parts along the implant plate element renders it possible on the one hand to fasten a bone screw to a high degree independently of the basic position of the plate element and, on the other hand, at a later stage to adjust the relative position of the plate element and the fastened bone screw. This can be utilized in an advantageous manner, for instance, in connection with the reduction of vertebrae.

In the above-mentioned known publication, the positions of the bone screws are adjusted by adjusting elements, which may be in the shape of a rod element, by means of which the angular position of a fastened bone screw relative to the plate element can be adjusted, being temporarily locked to the bone screw at issue by means of a threaded joint in an internal threaded bore in the bone screw. Adjusting rods are then fixed in the bone screws, whereupon an expanding tool is caused to engage the two adjusting rods. By means of the tool, the adjusting rods are forced apart, which means that the bone screws are straightened relative to the plate element during simultaneous changing of the position of the vertebrae, in which the bone screws are fastened.

A drawback of this technique of adjusting the positions of the bone screws is that it is complicated and necessitates a plurality of space-requiring adjusting tools, which renders an operation difficult.

### Objects of the Invention

One object of the present invention is to provide an implant device, by means of which increased flexibility in respect of the adjusting of the positions of the bone screws relative to the implant element is obtained.

One more object of the invention is to provide an implant device, by means of which increased possibilities of use are obtained.

### Summary of the Invention

According to the invention, the above-mentioned objects are achieved by an implant device of the type mentioned by way of introduction having the features as defined in the appended claims.

In accordance with a first aspect of the invention, an implant device thus is distinguished by a rotatably arranged adjusting means and transmission means adapted to transmit a rotary motion of the adjusting means to a translatory motion of the insert element in relation to the implant element.

According to a preferred embodiment of the invention, said adjusting means is arranged in connection with the implant element, preferably in a recess therein, and said transmission means comprises a gear wheel portion arranged on said adjusting means, and, coacting therewith, a rack portion arranged on said insert element and preferably integrated therewith.

By applying the invention, it is made possible to adjust, in an accurate and controlled fashion, the position of the bone screw holes in the insert element in relation to the implant device. The inventive adjusting means is advantageously operated by means of adjusting tools of a kind similar to that applied in key-hole surgery, whereby readjustment of the fastening of the implant device in the skeleton can be carried out without necessitating open surgery, which causes considerably less strain to the patient and thus constitutes a very great advantage.

A further advantage of the invention is that the position of the bone screw hole in the insert element in relation to the implant element can be fixed on the one hand by said transmission means holding the insert element in a controlled position according to the rotary position of the adjusting means during the actual adjusting operation and, on the other hand, by locking means being arranged to lock the rotary position of the adjusting means, and/or the position of the insert element, after adjustment.

The invention will now be described in more detail by means of an embodiment with reference to the accompanying drawings.

### Brief Description of the Drawings

Fig. 1 is a schematic top plan view of an implant according to prior art.

Fig. 2a is a schematic bottom view of an end portion of an implant device according to an embodiment of the invention.

Fig. 2b is a schematic top plan view of the end portion illustrated in Fig. 2a.

Figs 2c and 2d are separate schematic views from below and from above, respectively, of the insert element and the adjusting pin which are included in the implant device in Figs 2a and 2b.

Figs 3a, 3b and 3c are schematic cross-sections along line I-I extending through the implant device in Fig. 2a.

### Description of the Preferred Embodiment

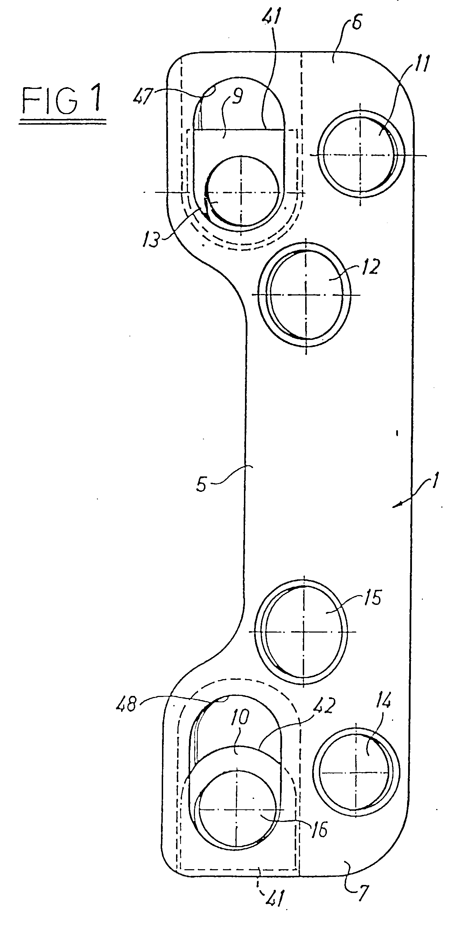

Fig. 1 illustrates an example of an implant according to prior-art technique. The implant comprises an implant element 1, insert elements 9, 10 and bone screws (not shown) used for fixing the implant in bones, such as vertebrae. The implant has six circular similar bone screw holes 11-16, four of which 11, 12, 14, 15 are fixedly arranged in the implant element 5 and two 13, 16 are displaceably arranged in the implant, one in each insert element 9, 10.

Each bone screw hole has a concave, spherically curved wall and is adapted to receive an annular hole insert (not shown) which has a convex circumferential surface which is curved in a correspondingly spherical manner, and a circular-cylindrical internal wall intended for coaction with the circular cylindrical main part of a bone screw. Each annular hole insert is designed such that a bone screw inserted therein is locked in a position in which it is no longer longitudinally displaceable in relation to the hole insert, but in which it can still rotate about its longitudinal axis relative to the hole insert.

As is evident from Fig. 1, the insert elements 9 and 10 are displaceable in alignment with the longitudinal axis of the implant element 1. The insert elements 9, 10 are arranged in corresponding recesses in the implant element 1, said recesses extending inwards from the respective terminal edges of the implant element 1.

To provide access to the bone screw holes 13, 16 in the insert elements 9, 10, holes 47, 48 are formed in the implant element above the insert element recesses. The holes 47, 48 are elongate with a width slightly greater than the diameter of the bone screw holes and with semicircular end portions, which in respect of position correspond to the two extreme positions of the holes 13, 16 of the plate parts.

An embodiment of an implant device according to the invention will now be described with reference to Figs 2a-2d and Figs 3a-3c. It will be appreciated that, in addition to that described below, the implant device may have a general configuration of the type as described for the implant in Fig. 1.

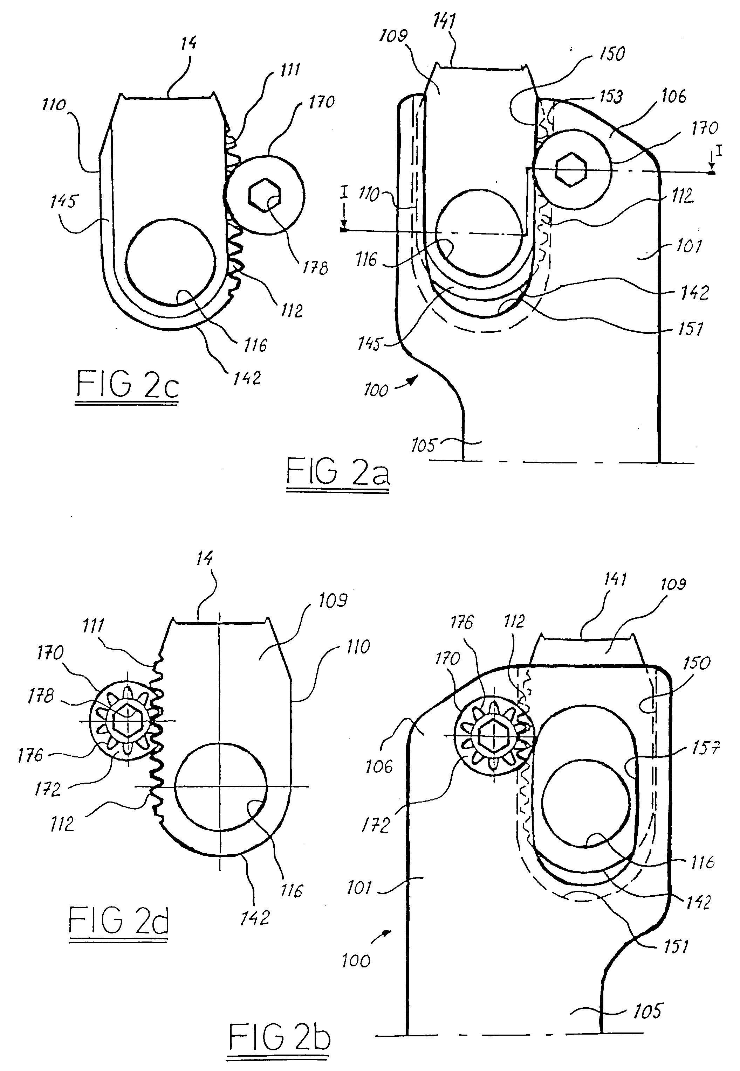

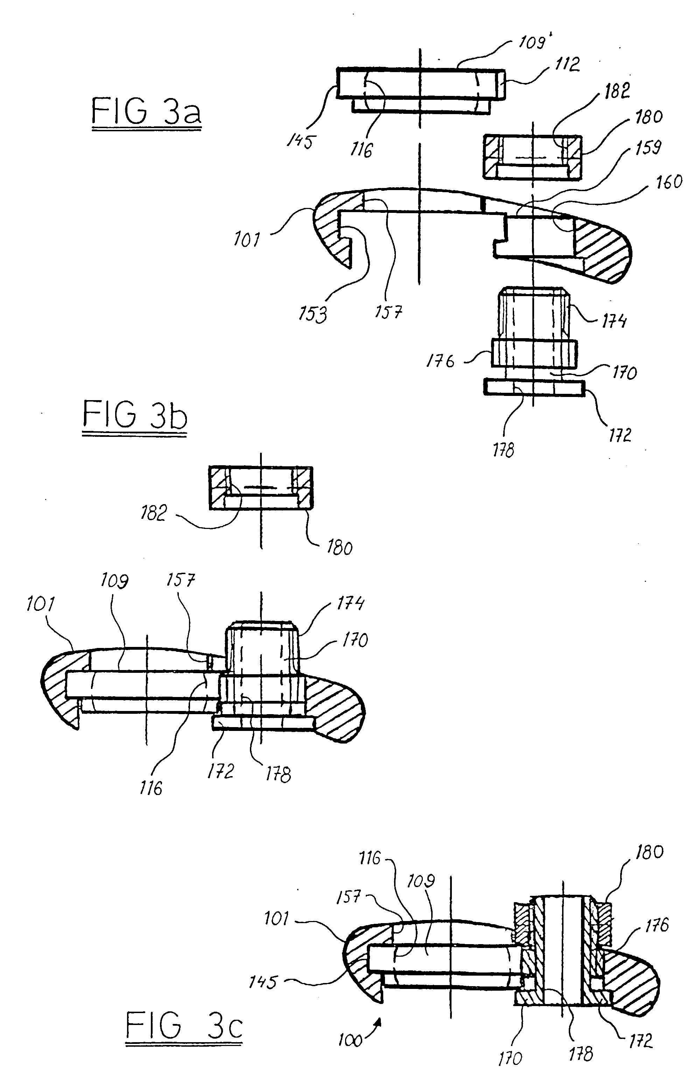

Figs 2a and 2b are schematic views from below and from above, respectively, of an end portion of an implant device 100 according to an embodiment of the invention, the not shown end portion of the implant device 100 advantageously being designed correspondingly. Furthermore, for easier understanding Figs 2c and 2d are schematic views from below and from above, respectively, of the insert element and the adjusting pin that are included in the implant device in Figs 2a and 2b. Finally, Figs 3a, 3b and 3c are schematic cross-sections along line I-I in Fig. 2a extending through the implant device in three different mounting positions.

The implant device comprises an implant element 101, which consists of a slightly arched elongate plate having a central part 105 with a reduced width and end parts 106. Each end part 106 accommodates a movable insert element 109. Moreover, the implant device has a number of identical bone screw holes for fixing of bone screws, one hole 116 being formed in the insert element 109.

As appears from Figs 2a and 2b, the insert element 109 and thus the bone screw hole 116 formed therein are displaceable in relation to the implant element 101 and parallel with the longitudinal axis of the implant element 101. The insert element 109 is flat and elongate and has two parallel lateral edges 110 and 111, a conically tapering and then straight cut-off end 141 and a part-circularly rounded end 142. One 111 of the parallel lateral edges of the insert element 109 has a number of teeth which are arranged along the lateral edge 111 and which together form a rack portion 112.

The insert element 109 is arranged in a recess 150 in the underside of the implant element 101, said recess 150 extending inwards from the terminal edge of the implant element 101 and having a rounded termination 151 in conformity with the rounded one end 142 of the insert element 109. The insert element 109 is inserted, rounded end 142 first, into the recess 150 from the end of the implant element 101 where the recess 150 is open.

The recess 150 has a guiding groove 153, which is obvious from Fig. 3a, in which a corresponding square guiding projection 145 of the insert element 109 engages and holds the insert element displaceable in place. The guiding projection 145 extends along the lateral edges 110 and 111 and the rounded terminal edge 142 of the insert element 109, the rack portion 112 on the lateral edge 111 constituting the extension of the guiding projection 145 on the lateral edge 111.

To provide access to the bone screw hole 116 in the insert element 109, an access hole 157 is formed in the implant element 101 above the insert element recess 150. The hole 157 is elongate with a width slightly greater than the diameter of the bone screw hole 116, but smaller than the distance between the circumference of the guiding projection 145 on the lateral edges 110 and 111, and has semicircular end parts, which in respect of position correspond to extreme positions of the hole 116 of the implant element 109, one position corresponding to the insert element 109 inserted into the recess 150 as far as possible, half the circumference of the bone screw hole 116 being aligned with the inner semicircular circumference of the access hole 157, the other corresponding to the insert element 109 displaced as far (functionally) outwards as possible, the other circumference half of the bone screw hole 116 being aligned with the outer semicircular circumference of the access hole 157.

Moreover, the implant element 101 has a recess in the form of a hole 160 which extends through the implant element and which communicates with the recess 150 and is adapted to receive an adjusting means in the form of a generally cylindrical adjusting pin 170. In one end, the adjusting pin 170 is provided with a stop flange 172 and in the other end with an external thread 174.

Further a central portion of the adjusting pin, between the stop flange 172 and the external thread 174, is provided with an external gear in the form of a gear wheel 176, the teeth of which are adapted to mesh with the teeth of the rack portion 112 of the insert element 109 when the adjusting pin 170 is inserted into the through hole 160 and the insert element 109 is inserted in the recess 150 with the lateral edge 111 which has the rack portion 112 facing the recess 160 for the adjusting pin 170, whereby turning or rotation of the adjusting pin 170 in the hole 160 and, thus, of the gear wheel 176 will cause the insert element 109 to be displaced in the longitudinal direction of the recess 150.

The stop flange 172 is adapted to abut against the underside of the implant element 101 at the circumference of the hole 160, when the adjusting pin 170 is arranged in the hole 160. The distance between the stop flange 172 and the gear wheel portion 176 is such that the central portion of the adjusting pin 170 with the gear wheel 176 is arranged on the same level as the guiding groove 153 in the recess 150, i.e. in level with the rack portion 112 of the insert element 109, when the adjusting pin 170 is arranged in place in the hole 160, as appears from Fig. 3b.

The external thread 174 of the adjusting pin 170 is adapted to receive a locking nut 180 having a corresponding internal thread 182, after the adjusting pin 170 has been arranged in place in the hole 160. The locking nut 180 is used to lock the adjusting pin 170 in a desired rotary position, after the desired adjustment of the position of the insert element 109, by the locking nut being fastened on the external thread 174 of the adjusting pin 170 to press against the upper side of the implant element 101, as is evident from Fig. 3c, thereby preventing further rotation of the adjusting pin 170 and, thus, further displacement of the insert element 109. It should be noted that the locking nut 180 need not be unscrewed completely from the thread 174 of the adjusting pin 170 to permit rotation of the adjusting pin 170 when adjusting the position of the insert element 109, but the locking nut 180 is advantageously screwed back only so far that the effect of the pressing against the upper side of the implant element 101 will be sufficiently small, alternatively ceases, to allow rotation of the adjusting pin 170.

The locking of the rotary position of the adjusting pin 170 and the translatory position of the insert element 109 in the recess 150 is achieved in three ways: by the pressing per se of the locking nut 180 against the upper side of the implant element; by the stop flange 172 of the adjusting pin being designed to have such a large diameter that, when the locking nut 180 is tightened to be pressed against the upper side of the implant element 101, the stop flange is pressed against the actual insert element 109 and thus locks this; by that surface of the upper side of the implant element 101 against which the locking nut 180 is caused to be pressed, having an angle in relation to the axial direction of the adjusting pin 170 which deviates slightly from 90°, whereby the pressing of the locking nut 180 against the upper side of the implant element 101 results in a small inclination of the adjusting pin in the hole 160, which further increases the locking of the adjusting pin.

For enabling the adjusting pin to be inserted into the hole 160 in a position when the insert element 109 is already arranged in the recess 150 with teeth of the rack portion 112 extending from the recess 150 into the hole 160 and partially blocking the same, spline-like discontinuities in the thread (not shown), which in respect of position coincide with the troughs between the teeth of the gear wheel 176, are arranged in the external thread 174 of the adjusting pin 170.

Furthermore, the adjusting pin 170 has a centrally arranged through hole 178 in the form of an Allen wrench groove, extending in the axial direction of the pin. This through hole is adapted to be used for temporary engagement of adjusting tools when actuating the adjusting pin 170, which thus can easily be fixed to and released from the adjusting pin 170.

Although the invention has been described above with reference to a preferred, exemplifying embodiment thereof, it will be appreciated that various alterations and modifications can be carried out within the scope of protection of the invention, which is defined by the appended claims.

## Claims

1. An implant device comprising an implant element (101) and an insert element (109) which is translatorily movable in the implant element, said insert element having a bone screw hole (116) adapted to receive a bone screw which is insertable and lockable therein, and movement of the insert element (109) in relation to the implant element (101) giving adaption of the position of the bone screw hole (116) in relation to the implant element (101), **characterized** by
a rotatably arranged adjusting means (170) and transmission means (112, 176) adapted to transmit a rotary motion of the adjusting means (170) to a translatory motion of the insert element (109) in relation to the implant element (101).

2. An implant device as claimed in claim 1, wherein said adjusting means (2) is arranged in or on the implant element (101).

3. A device as claimed in claim 1 or 2, wherein said adjusting means (170) is rotatably arranged in a recess (160) in said implant element (101).

4. A device as claimed in claim 1, 2 or 3, wherein said transmission means comprises a gear wheel portion (112) arranged on said adjusting means (117), and, coacting therewith, a rack portion (112) arranged on said insert element (109) and preferably integrated therewith.

5. A device as claimed in claim 4, wherein said rack portion (112) is formed on an edge or side (111) of said insert element (109) extending in the longitudinal direction of the implant element (101).

6. A device as claimed in claim 4 or 5, wherein said adjusting means is formed as a pin (170) preferably extending through the implant element (101), said gear wheel portion (176) being formed on the circumference of said pin.

7. A device as claimed in claim 6, wherein said pin (170) comprises a threaded portion (174) designed to receive a locking nut (180), which is intended for releasable locking of the rotary position of the pin (170), preferably by being pressed against the surface of the implant element (101).

8. A device as claimed in claim 7, wherein said adjusting means (170) is arranged in a recess (160) formed in the implant element (101) and communicating with said elongate recess (150).

9. An implant device as claimed in claim 1, wherein said adjusting means is arranged on the insert element (109), and said transmission means comprises a gear wheel portion arranged on said adjusting means, and a rack portion coacting with the gear wheel portion and arranged on the implant element (101), preferably integrated therewith.

10. A device as claimed in any one of the preceding claims, wherein said insert element (109) is movable in an elongate recess (150) formed in the implant element (101).

11. A device as claimed in any one of the preceding claims, wherein said adjusting means (170) comprises means (178) for temporary attachment of adjusting tools for actuating said adjusting means.

12. A device as claimed in claim 10, wherein said means for temporary attachment consists of a key recess (178) formed in said adjusting means, preferably in one end thereof.

13. A device as claimed in any one of the preceding claims, **characterized** by locking means (180) for preferably releasable locking of the rotary position of said adjusting means (170) and/or the position of said insert element (109) after adjustment as desired.

14. A device as claimed in any one of the preceding claims, wherein said insert element (109) is movable in the longitudinal direction of the implant element (101).

15. A device as claimed in any one of the preceding claims, wherein said insert element (109) is a substantially plate-like element.

16. A device as claimed in any one of the preceding claims, wherein said implant element (101) is a substantially plate-like element.
